# EUROPEAN PATENT SPECIFICATION

(11) **EP 4 490 260 B1**
(45) Date of publication and mention of the grant of the patent: **10.12.2025**
(21) Application number: 23714827.5
(22) Date of filing: 07.03.2023
(51) Int. Cl.: C12M 1/00, C12M 1/06, C12M 1/34

(54) **BIOREACTOR FOR PRODUCING MICRO-ORGANISMS AND RESPECTIVE PRODUCING METHOD**
BIOREAKTOR ZUR HERSTELLUNG VON MIKROORGANISMEN UND ENTSPRECHENDES HERSTELLUNGSVERFAHREN
BIORÉACTEUR POUR LA PRODUCTION DE MICRO-ORGANISMES ET PROCÉDÉ DE PRODUCTION RESPECTIF

(30) Priority: 07.03.2022 IT 202200004289
(43) Date of publication of application: 15.01.2025
(73) Proprietor: Wheel-e S.r.l., 00046 Grottaferrata (RM) (IT)
(72) Inventor: DI GIUSEPPE STEFANORI, Davide, 00046 Grottaferrata (RM) (IT); SBARDELLA, Euro, 00046 Grottaferrata (RM) (IT)
(74) Representative: Ercolani, Simone Pietro
(86) International application number: PCT/IB2023/052119
(87) International publication number: WO 2023/170561

(56) References cited:
- EP-A1- 0 164 811
- EP-A1- 0 889 118
- WO-A1-99/01537
- US-A- 4 406 162
- US-A- 5 226 747

## Description

### FIELD OF THE INVENTION

The present invention concerns a bioreactor for producing micro-organisms and respective method. In particular, this bioreactor is adapted to obtain microalgal and bacterial biomasses for the food, nutraceutical, pharmaceutical, cosmetic and zootechnical sectors.

### KNOWN PRIOR ART

The existence of bioreactors for producing such micro-organisms has long been known. In fact, industrial microbiology processes take place mainly within bioreactors, or even known as fermenters, whose technological development has enabled significant advances in the scope of applied microbiology. The best known fermenter is a reactor consisting of a vertical steel tank. This type of fermenters originated and evolved from analogous plants used in chemistry, whose industrial application is earlier than microbiology.

The technological characteristics of the plant and, in particular, of the fermenter depend on the metabolism of the micro-organism involved which, depending on the physiology, may require aerobic or anaerobic conditions. The dimensions of the fermenter vary depending on the process and range from a few tens of m³, e.g., for producing some biopharmaceuticals, up to 1000 m³, e.g., for producing ethanol. At the top of the fermenter, or bioreactor, there is a series of inlets and outlets which are controlled by solenoid valves. The inlets are needed for the inoculation, inside the bioreactor, of the liquid culture and any additions such as, e.g., nutrilites, pH correctors, anti-foam agents and the like, while the outlets are used for spent air and any culture samples carried out over time to monitor the process.

The temperature inside the fermenter is maintained at optimal levels in relation to the needs of the micro-organism by means of internal or external devices. In addition, the liquid culture inside the bioreactor is kept under stirring to homogenise the environment, thus avoiding the formation of gradients related not only to temperature but also to cell biomass, nutrilites and gases present in the same liquid culture. Stirring devices have different design depending on the characteristics of the process, in particular, of the cell macromorphology. Nevertheless, in general, the stirring device consists of a drive shaft to which the impellers are constrained and which extends vertically along the longitudinal axis of the bioreactor's tank. Among the fast agitators, which have an agitator diameter to reactor diameter ratio and can be used with unicellular micro-organisms (e.g. yeasts and most bacteria), the most common are marine propeller, paddle and turbine agitators, such as, e.g., of the *Rushton* type. Among the slow agitators, which are characterised by an agitator diameter to reactor diameter ratio and can be used with mycelial micro-organisms (e.g., moulds and some bacteria), the most common are cage, anchor and worm agitators.

However, these tank bioreactors are not without drawbacks. In fact, stirring devices, when used at high speeds, in order to obtain a higher production of micro-organisms, result in the foam being formed on the surface of the culture, the disruption of cells due to the shear forces produced by the impeller and cell lysis with the leakage of extracellular products into the fermentation broth, consequently causing obvious damage to the metabolism and growth of the micro-organisms present in the liquid culture which is contained in the bioreactor or fermenter.

Parallel to these types of tank bioreactors, the bioreactors of the wave-induced type have long been developed, mainly used for photosynthetic micro-organisms. For example, the patent WO99/01537, in the name of INGREDIENT TECHNOLOGY CORPORATION INTERNATIONAL, describes a bioreactor of this kind.

This type of bioreactor comprises a reaction channel containing a liquid culture of said micro-organisms and means of generating, in said liquid culture, harmonic gravity waves propagating in the direction of the longitudinal axis of said channel. These means of generating harmonic waves include, in a known way, a blade that rotates alternately around a hinge placed at the bottom of the channel in such a way as to generate harmonic gravity waves in the same liquid culture. On the top, such a bioreactor has a transparent lid to protect the liquid culture but, at the same time, allows the passage of light radiation.

It should be noted that the above-mentioned harmonic gravity waves have dynamics similar to waves present in the open sea. In this type of waves, gravity tends to flatten the wave crests and restore equilibrium. The excess water that appears at the wave crest must flow out of the regions of adjacent grooves, so the single particles are subject to a motion that is the combination of the longitudinal motion, back and forth, and the transverse motion, up and down, of the same wave. The single floating cells in a harmonic wave of this kind do not undergo any mean translation and move along circular, or elliptical, trajectories depending on the frequency of the same wave. Thus, once a wave propagates along the longitudinal direction of the channel, the generic particle, or cell, of the micro-organism present in the liquid culture is subjected to a motion along circular, or elliptical, trajectories, but is not dragged along the longitudinal axis of the channel and returns back, after a period of time, into the same starting position.

However, these circular and elliptical trajectories are not sufficient to obtain the maximum productivity in terms of microalgal biomass because of the reflections that occur, especially when increasing the length scale of the growth channel of these micro-organisms.

The inclined plane, as per known art, was also found to be insufficient to keep the equilibrium state of the wave train within the growth channel, thus failing to completely prevent the reflection phenomenon at lengths greater than two metres.

It is also known that in order to obtain a high increase in photosynthesis, it is necessary that within these standing harmonic waves, there can be a total rotation of the algal cells present in the culture liquid, from the bottom up to the crest of the wave profile, so as to achieve uniformly adequate exposure to light, thus improving the same algal growth.

However, these conditions on larger scales are diminished, due to the phenomenon of reflections of the waves produced, which significantly reduces the growth rate of photosynthetic micro-organisms and therefore, ultimately, the productive capacity of the bioreactor. It was also found that the reflection component, if present, also damages the exchange of gradients, such as, e.g., the micro-elements and CO2 that are needed for the development of these photosynthetic micro-organisms, including the heterotrophic, autotrophic and chemosynthetic ones.

The document US4406162A describes an apparatus for creating surface waves in a liquid that comprises a liquid displacer, a motor and a linear actuator to impart movement to the displacer and a level sensing device cooperating with the displacer to control the liquid level through the control of the generated wave. This apparatus is used to perform wave tests to simulate the aquatic conditions for ships, oil platforms and port installations.

Therefore, object of the present invention is to implement a wave-induced type bioreactor and a respective method capable of overcoming the problems mentioned above and, therefore, significantly increasing the production levels of the micro-organisms present in the liquid culture which is contained therein, both photosynthetic and aerobic and/or heterotrophic ones.

Furthermore, object of the present invention is to implement the above in a simple way and without making excessive modifications to wave-induced bioreactors of known art.

Finally, object of the present invention is also to implement an implementable method for the operation of the apparatus according to the invention.

### SUMMARY OF THE INVENTION

These and other objects are achieved by means of a bioreactor for the production of photosynthetic micro-organisms comprising a reaction channel containing a liquid culture of said micro-organisms and generating means for generating waves which propagate in the direction of the longitudinal axis of said reaction channel, starting from an input command signal to said generating means in which the shape of the harmonic gravity waves intended to be generated along the longitudinal axis of said reaction channel, is depicted, said generating means comprising at least one blade movably constrained within said reaction channel, at a first end of said channel, to generate said longitudinal propagation waves, said bioreactor being characterised by also comprising detecting means for detecting the component of the reflected waves which is contained in said longitudinal propagation waves produced by said generating means, and regulating means for dynamically regulating the displacement frequency and/or amplitude of said at least one blade depending on the information received from said detecting means, in such a way as to zero out the component of the reflected waves which is contained in said longitudinal propagation waves to obtain the shape of said harmonic gravity waves intended to be generated along the longitudinal axis of said reaction channel.

This solution, obviously, allows to solve the problems of the bioreactors of known art. In fact, it is possible to continuously and dynamically zero out the component of the reflected waves present in the generated wave so as to obtain the shape of the harmonic gravity waves (i.e. standing waves) generated along the longitudinal axis of the reaction channel.

According to a first embodiment of the invention, said detecting means comprise first measuring means arranged within said reaction channel for detecting over time the shape of the longitudinal propagation waves generated by said generating means, wherein said regulating means dynamically regulate the displacement frequency and/or amplitude of said at least one blade depending on the information received from said first measuring means by comparing the shape of said longitudinal propagation waves generated by said generating means and detected by said first measuring means, with the shape of said harmonic gravity waves intended to be generated by said generating means, in such a way that the wave generated by said generating means substantially coincides with the wave intended to be generated by said generating means.

In particular, said first measuring means comprise at least two level sensors capable of measuring over time the level variation of said longitudinal propagation waves generated by said generating means within said liquid culture and of sending said information to said regulating means. Said at least two level sensors are arranged in two distinct positions of space along said longitudinal direction of said channel. Preferably, said at least two level sensors are arranged at an identical height.

In a second embodiment of the invention, said detecting means comprise second measuring means arranged on said at least one blade for detecting, over time, the hydrodynamic forces acting on said at least one blade; said regulating means dynamically regulate the displacement frequency and/or amplitude of said at least one blade depending on the information received by said second measuring means. Furthermore, the objects are also achieved by a method for producing photosynthetic micro-organisms by means of a bioreactor according to one or more of claims 1 to 11, comprising the step of:
a) generating, by means of said generating means, propagation waves along the longitudinal axis of said reaction channel containing a liquid culture of said micro-organisms, said step a) being carried out starting from an input command signal to said generating means in which the shape of the harmonic gravity waves intended to be generated along the longitudinal axis of said reaction channel, is depicted;
characterised by comprising the steps of:
b) detecting the component of the reflected waves which is contained in said longitudinal propagation waves produced by said generating means;
c) dynamically regulating the displacement frequency and/or amplitude of said at least one blade depending on the information received during the step b) in such a way as to zero out said component of the reflected waves which is contained in said longitudinal propagation waves to obtain the shape of said harmonic gravity waves intended to be generated along the longitudinal axis of said reaction channel.

In particular, said step b) comprises the step b1) of comparing the shape of said longitudinal propagation waves generated by said generating means in said step a), with the shape of said harmonic gravity waves intended to be generated by said generating means, in such a way that the longitudinal propagation wave generated by said generating means substantially coincides with the wave intended to be generated by said generating means.

According to a first embodiment of the invention, said step b1) comprises the step b1a) of detecting the level variations of the free surface of said liquid culture, over time, in at least two distinct points arranged along said longitudinal axis of said reaction channel, thus obtaining a plurality of pairs of level variations at different instants, wherein said two distinct points are measured by a first origin point of generation said harmonic gravity waves by said at least one blade, the step b1b) of adding, for each detection carried over time, the two detected level variations, thus obtaining a plurality of sums of level variations, and the step b1c) of subtracting, for each detection, said plurality of sums of such level variations from the sum of two level variations of the shape of said harmonic gravity waves intended to be generated, taken in two points arranged at a distance, from a second origin, equal to that at which said two distinct points are located with respect to said first origin point of said propagation waves generated by said at least one blade.

Finally, said step c) comprises the step c1) of varying the displacement frequency and/or amplitude of said at least one blade if said difference calculated in said step b1c) is different from zero.

Again, according to a second embodiment of the invention, said step b) comprises the step b1') of detecting over time the hydrodynamic forces acting on said at least one blade, and the step b2') of subtracting for each instant the force measured with the force value expected at that given instant; said step c) further comprises the step c1') of varying the displacement frequency and/or amplitude of said at least one blade if said difference calculated in said step b2') is different from zero.

In both embodiments described above, it emerges that in said step c1), or c1'), said variation in frequency and/or amplitude is obtained through a transfer function which uses said value calculated in said step b1c), or b2'), as input signal, and that it is adapted to feedback minimise said difference value calculated in said step b1c), or b2'), through a closed-circuit control system of the PID type or neural networks or fuzzy logic or the like.

These and other aspects of the present invention will be made clearer by the following detailed description of a preferred embodiment provided herein only by way of non-limiting example, with reference to the accompanying figures, wherein:
Figure 1A is a view of a bioreactor of known art;
Figure 1B is a simplified side view of the bioreactor of figure 1A in which the theoretical movement of micro-organisms is shown within the liquid culture in the case of harmonic gravity waves;
Figure 2 is a simplified longitudinal view of a first embodiment of the invention;

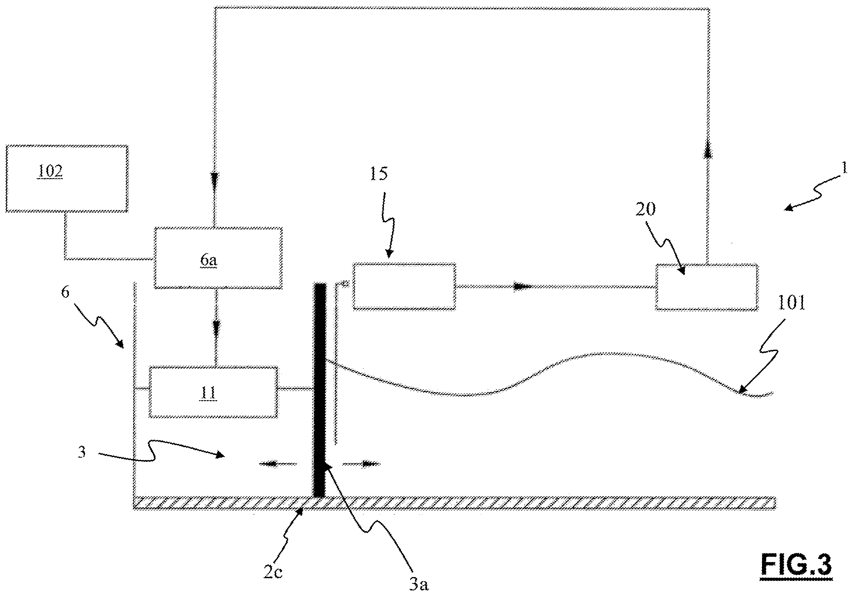

Figure 3 is a simplified longitudinal view of a second embodiment of the invention;
Figure 4A is a view of the harmonic gravity wave intended to be generated by the generating means of the bioreactor;
Figure 4B is a view of the longitudinal propagation wave generated by the generating means of the bioreactor.

### DETAILED DESCRIPTION OF A PREFERRED EMBODIMENT OF THE PRESENT INVENTION

With particular reference to these figures, 1 denotes a bioreactor according to the invention.

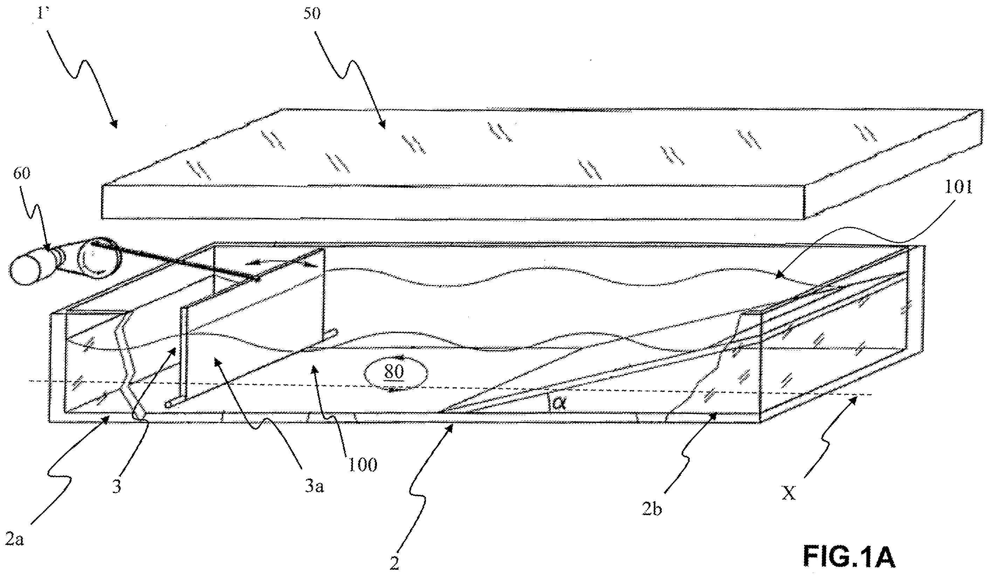

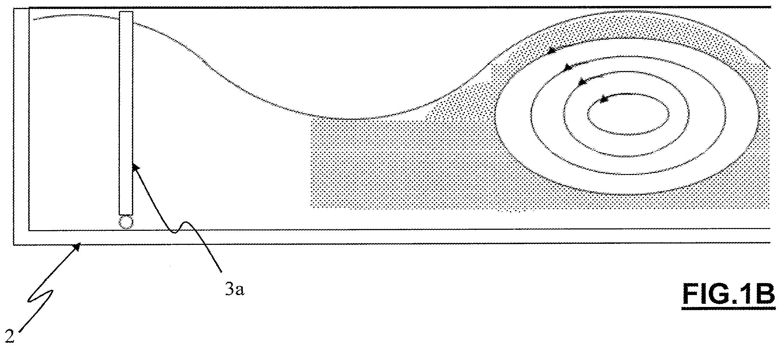

According to known art, as shown in figure 1, the bioreactor 1' for producing photosynthetic micro-organisms comprises a reaction channel 2 containing a liquid culture 80 of micro-organisms and generating means 3 for generating waves 101 which propagate in the direction of the longitudinal axis X of the reaction channel 2, starting from an input command signal to such generating means 3 in which the shape of the harmonic gravity waves 102 intended to be generated along the longitudinal axis X of the reaction channel 2, is depicted. These generating means 3 comprise a blade 3a movably constrained within the reaction channel 2, at a first end 2a of the channel 2, for generating the above-mentioned longitudinal propagation waves 101. The bioreactor 1' also comprises a transparent lid 50 for covering the reaction channel 2. In addition, this bioreactor 1' also comprises a motor 60 adapted to control the displacement of the blade 3a.

The frequency of these propagation waves, which have a substantial sinusoidal shape, is between 0.1 and 3 Hz. In the present case, the frequency of the propagation waves 101 is 1 Hz, however in other examples this frequency may also be different from that specified herein and, in any case, in the range between 0.1 and 3 Hz, without thereby departing from the protection scope of the present invention.

Figure 1B shows the rotational movement undergone by the micro-organisms within the liquid culture once the generating means 3 start working by generating waves 101 which propagate in the direction of the longitudinal axis X of the reaction channel 2.

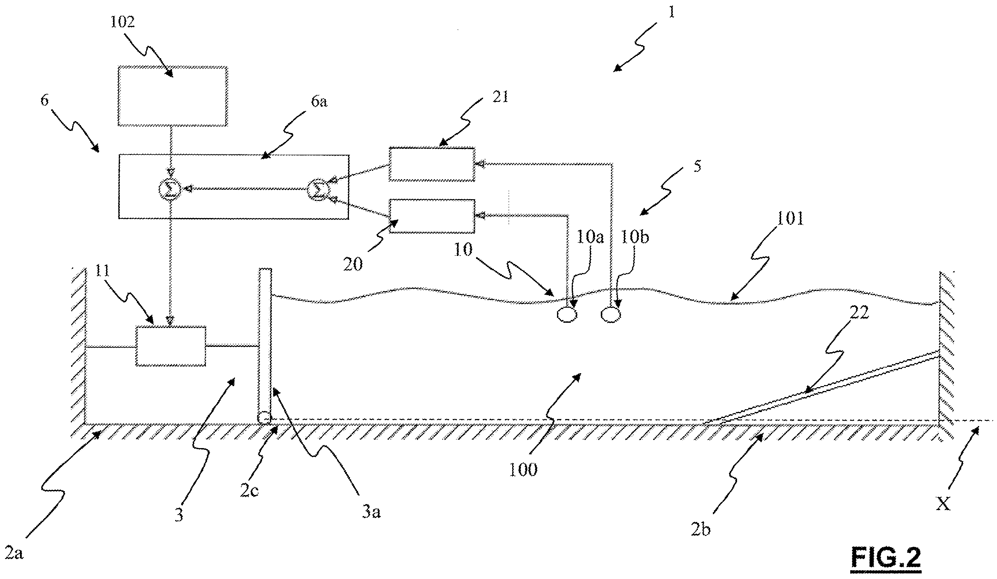

According to the invention, as shown in figure 2, the bioreactor 1, which for the same elements present in the bioreactor 1' of known art of figure 1 has identical references, comprises a reaction channel 2 containing a liquid culture 100 of micro-organisms such as, e.g., Dunaliella bardawil, Spirulina platensis, Isochrysis galbana and generating means 3 to generate waves 101 propagating in the direction of the longitudinal axis X of the reaction channel 2, starting from an input command signal to such generating means 3 in which the shape of the harmonic gravity waves 102 intended to be generated along the longitudinal axis X of the reaction channel 2, is depicted. These generating means 3 comprise a blade 3a movably constrained within the reaction channel 2, at a first end 2a of the channel 2, for generating the above-mentioned longitudinal propagation waves 101. The bioreactor 1 also comprises a transparent lid (not shown herein but quite similar to the lid 50 of figure 1A) for covering the reaction channel 2.

Advantageously, the bioreactor 1 also comprises means 5 for detecting the component of the reflected waves which is contained in the longitudinal propagation waves 101 produced by the generating means 3 and regulating means 6 for dynamically regulating the displacement frequency and/or amplitude of the blade 3a depending on the information received by the detecting means 5, in such a way as to zero out the component of the reflected waves which is contained in the longitudinal propagation waves 101 to obtain the shape of the harmonic gravity waves 102 intended to be generated along the longitudinal axis X of the reaction channel 2.

According to the embodiment described herein, the detecting means 5 comprise the first measuring means 10 arranged within the reaction channel 2 for detecting over time the shape of the longitudinal propagation waves 101 generated by the generating means 3. These regulating means 6 regulate the displacement frequency and/or amplitude of the blade 3a dynamically depending on the information received from the first measuring means 10 by comparing the shape of the longitudinal propagation waves 101 generated by the generating means 3 and detected by the measuring means with the shape of the harmonic gravity waves 102 intended to be generated by the generating means 3, in such a way that the wave generated by the generating means 3 substantially coincide with the wave intended to be generated by the generating means 3.

In particular, the first measuring means 10 comprise two level sensors 10a,10b capable of measuring over time the level variation of longitudinal propagation waves 101 generated by the generating means 3 within the liquid culture.

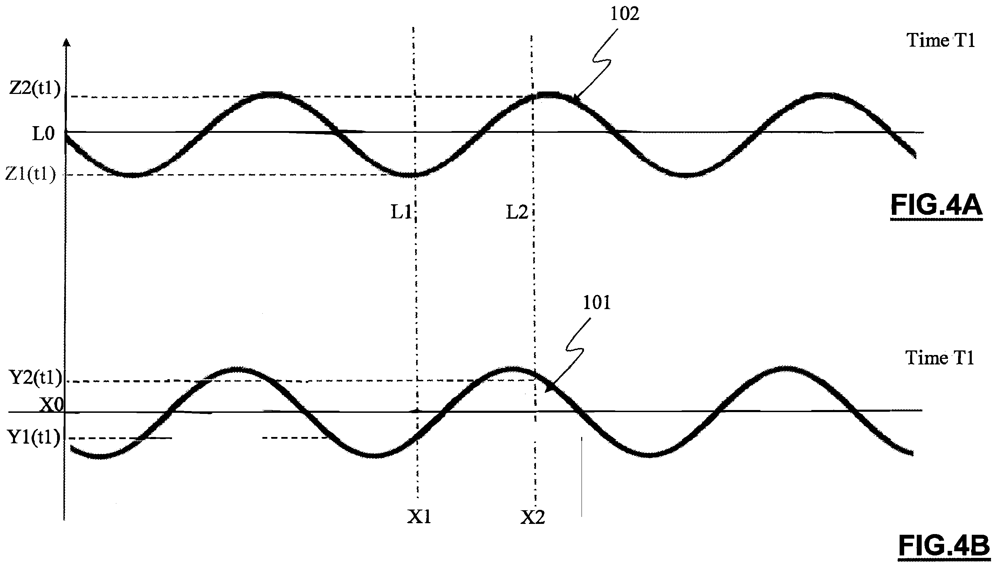

Furthermore, the regulating means 6 comprise a control unit 6a adapted to receive the information obtained from the first measuring means 10 to process it. This control unit 6a is then capable of controlling the displacement frequency and/or amplitude of the blade 3a depending on the results obtained by the control unit 6a when processing the information received by the two sensors 10a,10b. In fact, the first measuring means 10 send the information obtained to the control unit 6a. The two level sensors 10a,10b are arranged at the same height but in two distinct positions of space X1,X2 along the longitudinal direction of said channel X (see figure 4B). Obviously, the height at which said level sensors 10a,10b are positioned is selected in such a way that it is possible to detect the longitudinal propagation wave 101 which is formed within the reaction channel 2.

It should be emphasised that the number of level sensors 10a,10b may also be higher than two, without thereby departing from the protection scope of the present invention. These level sensors 10a,10b are of the piezoelectric type. In another embodiment, these sensors 10a,10b may also be of optical, inductive or capacitive type or other types, without thereby departing from the protection scope of the present invention.

Still according to the embodiment described herein, the blade 3a is vertical and is rotatably constrained to the base 2c of the reaction channel 2.

Again, the dynamic regulating means 6 comprise a motor or an actuator 11 for displacing the blade 3a. The motor 11, or the actuator, is functionally connected, on the one side, to the control unit 6a and, on the other side, to the blade 3a, in order to be controlled to transmit the displacement control to the blade 3a according to a frequency and/or an amplitude calculated depending on the result obtained by the control unit 6a.

The motor 11 will therefore transform a rotary motion (that of the same motor) into a reciprocating rotary motion of the blade 3a. The parameter, therefore, of interest for the motor 11, i.e., the one that is dynamically varied, in a continuous manner, so that the component of the reflected waves is minimized as much as possible, turns out to be the rotation speed of the same motor 11. This rotation speed will be regulated by the control unit 6a which receives the signals as input from the two level sensors 10a,10b and that provides the value of the speed of the electric motor 11 as output, which will tend to minimise the error between the shape of the wave 101 generated by the blade 3a and the expected shape of the wave 102 along the longitudinal axis X of the reaction channel 2.

This error is, in particular, used as input datum of a closed control system present in the control unit 6a, e.g. of the proportional, integrative, derivative (PID) type. Alternatively, neural network or fuzzy logic control systems could be used equivalently, without thereby departing from the protection scope of the present invention.

Furthermore, the bioreactor 1 comprises two filtering elements 20,21 of the signal coming from the first measuring means 10 to the control unit 6a. These filters are of the band-pass type, which is a low-pass, since the frequency range is between 0.1 and 3 Hz. The filter is used to eliminate any noise present in the signal, coming from the sensors 10a, 10b and can be implemented both as hardware in the acquisition card or as software within the control algorithm.

According to this embodiment of the invention, the reaction channel 2 comprises an inclined wall 22 arranged at the end 2b of the reaction channel 2, opposite the end 2a in which the blade 3a is arranged. The purpose of this inclined plane is to reduce the reflected waves generated by the generating means 3. In fact, the main purpose of this inclined wall 22 is to cause counterthrust in order to keep the standing generated waves in equilibrium.

In accordance with a further embodiment of the invention, these detecting means 5 comprise second measuring means 15 arranged on the blade 3a for detecting over time the hydrodynamic forces acting on the blade 3a. These regulating means 6 dynamically regulate the displacement frequency and/or amplitude of the blade 3a depending on the information received from the second measuring means 5.

In particular, the second measuring means 15 comprise a force sensor.

The control unit 6a, which receives the information as input from these second measuring means 15, compares the hydrodynamic force acting on the blade 3a as time changes, by comparing it with the expected force in the case of perfectly harmonic gravity waves. Any difference results in the error which is then used as input datum of a closed control system, e.g., of the proportional, integrative, derivative (PID) type. This control system is present in the control unit 6a.

Alternatively, neural network or fuzzy logic control systems could be used equivalently, without thereby departing from the protection scope of the present invention. This control system then allows command instruction to be sent to the motor 11 which, in turn, will actuate the blade 3a in order to reduce the error measured by the control unit 6a. Also in this case, the parameter of the motor 11, which is controlled by the control unit 6a and which is dynamically varied in a continuous manner, so that the component of the reflected waves is minimised as much as possible, turns out to be the rotation speed of the same motor 11.

Finally, in this embodiment, the blade 3a is wedge-shaped and is translatably constrained to the base 2c of the reaction channel 2. In addition, there is only one filter 20 instead of two, as in the preceding embodiment.

Even in this embodiment, the second measuring means 15 are functionally connected to the control unit 6a and comprise signal filtering elements therebetween.

The bioreactor described above operates according to the method illustrated herein below for producing photosynthetic micro-organisms.

This method comprises the steps of:
a) generating, by means of the generating means 3, propagation waves along the longitudinal axis X of the reaction channel 2 containing a liquid culture of micro-organisms 80, e.g., the photosynthetic micro-organisms such as micro-algae and bacteria, heterotrophic micro-organisms, chemosynthetic micro-organisms; this step a) is carried out starting from an input command signal to the generating means 3 in which the shape of the harmonic gravity waves 102 intended to be generated along the longitudinal axis X of the reaction channel 2, is depicted;
b) detecting the component of the reflected waves which is contained in the longitudinal propagation waves produced by the generating means 3;
c) dynamically regulating the displacement frequency and/or amplitude of the blade 3a depending on the information received during the step b) in such a way as to zero out the component of the reflected waves which is contained in the longitudinal propagation waves 101 to obtain the shape of the harmonic gravity waves 102 intended to be generated along the longitudinal axis of the reaction channel 2.

In particular, step b), in case the above-mentioned first measuring means 10 are used, comprises the step b1) of comparing the shape of the longitudinal propagation waves 101 generated by the generating means 3 during step a) with the shape of the harmonic gravity waves 102 intended to be generated by the generating means 3, in such a way that the longitudinal propagation wave generated by the generating means 3 substantially coincides with the wave intended to be generated by said generating means 3.

Going into greater detail, step b1) comprises step b1a) of detecting over time, in at least two distinct points X1, X2 arranged along the longitudinal axis X of the reaction channel 2, the level variations Y1(t), Y2(t) of the free surface of the liquid culture, thus obtaining a plurality of pairs of level variations Y1(t1), Y2(t1); Y1(t2), Y2(t2);...; Y1(tn), Y2(tn) at different instants t1,t2,...,tn. These two distinct points X1 and X2 are measured from a first origin point X0 for generating harmonic gravity waves by the blade 3a.

Furthermore, the step b1) also comprises the step b1b) of adding, for each detection carried out over the time t1, t2,..., tn, the two detected level variations, thus obtaining a plurality of sums of level variations Y1(t1)+Y2(t1); Y1(t2)+Y2(t2);......; Y1(tn)+Y2(tn). In case the waves 102 generated by the blade 3a were perfectly standing, i.e. harmonic gravity waves, this sum should remain constant over time, however the wave 101 generated, due to the reflected waves, will never be perfectly standing, so this sum will vary slightly over time.

Furthermore, following the step b1b), the step b1) also comprises the step b1c) of subtracting, for each detection or plurality of detections, the above-mentioned plurality of sums of these level variations Y1(t1)+Y2(t1), Y1(t2)+Y2(t2),......, Y1(tn)+Y2(tn) from the sum of the two level variations Z1(t1)+Z2(t1); Z1(t2)+Z2(t2);......; Z1(tn)+Z2(tn) of the shape of the harmonic gravity waves intended to be generated, which are taken at two points L1, L2 arranged at a distance, with respect to a second origin L0, equal to that at which the two distinct points X1,X2 are located with respect to the first origin point X0 of the propagation waves 101 generated by the blade 3a (see figures 4A and 4B).

At this point, the step c) comprises the step c1) of varying the displacement frequency and/or amplitude of the blade 3a if the difference calculated in the step b1c) is different from zero. This difference value is also known as system error.

During the step c1), the variation sought in displacement frequency and/or amplitude of the blade 3a is obtained through a transfer function which uses the difference value, or error, calculated in step b1c) as input signal, and which is adapted to feedback minimise the above-mentioned value calculated in said step b1c) through a closed-circuit control system of the PID type or neural networks or fuzzy logic or the like.

Over time, the difference between the above-mentioned two sums will always be continuously different from zero and, therefore, the control system will continually try to minimise this difference, which will then tend towards zero.

In the case of use of the second measuring means 15, the step b) comprises step b1') of detecting over time the hydrodynamic forces acting on the blade 3a and the step b2') of subtracting, for each instant, the force measured from the force value expected at that given instant. The step c) also comprises the step c1') of varying the displacement frequency and/or amplitude of the blade 3a if this difference, or system error, calculated during step b2'), is different from zero.

During the step c1'), the variation in displacement frequency and/or amplitude of the blade 3a with respect to the value previously input to the means 3 for generating the displacement of the blade 3a is obtained through a transfer function which uses the value calculated in the step b2'), i.e. the system error, as input value and which, through a closed-circuit control system of the PID type or neural networks or fuzzy logic or similar, it is capable of feedback minimising said value calculated in said step b2').

## Claims

1. Bioreactor (1) for producing photosynthetic micro-organisms, comprising
a reaction channel (2) containing a liquid culture (100) of said micro-organisms and generating means (3) for generating waves (101) which propagate in the direction of the longitudinal axis (X) of said reaction channel (2), starting from an input command signal to said generating means in which the shape of the harmonic gravity waves (102) intended to be generated along the longitudinal axis (X) of said reaction channel (2) is depicted, said generating means (3) comprising at least one blade (3a) movably constrained within said reaction channel (2), at a first end of said channel (2a), to generate said longitudinal propagation waves (101), said bioreactor (1) being **characterised by** also comprising detecting means (5) for detecting the component of the reflected waves which is contained in said longitudinal propagation waves produced by said generating means, and regulating means (6) for dynamically regulating the displacement frequency and/or amplitude of said at least one blade (3a) depending on the information received from said detecting means, in such a way as to zero out the component of the reflected waves which is contained in said longitudinal propagation waves (101) to obtain the shape of said harmonic gravity waves (102) intended to be generated along the longitudinal axis of said reaction channel (2).

2. Bioreactor according to claim 1, **characterised in that** said detecting means (5) comprise first measuring means (10) arranged within said reaction channel (2) for detecting over time the shape of the longitudinal propagation waves generated by said generating means (3), said regulating means (6) dynamically regulating the displacement frequency and/or amplitude of said at least one blade (3a) depending on the information received from said first measuring means (10) by comparing the shape of said longitudinal propagation waves (101) generated by said generating means (3) and detected by said first measuring means (10), with the shape of said harmonic gravity waves (102) intended to be generated by said generating means (3), in such a way that the wave generated by said generating means substantially coincides with the wave intended to be generated by said generating means.

3. Bioreactor (1) according to claim 2, **characterised in that** said first measuring means (10) comprise at least two level sensors (10a, 10b) capable of measuring over time the level variation of said longitudinal propagation waves generated by said generating means (3) within said liquid culture, and sending said information to said regulating means (6), said at least two level sensors (10a, 10b) being arranged in two distinct positions of the space (X1, X2) along said longitudinal direction of said channel (X).

4. Bioreactor (1) according to one or more of claims 1 to 3, **characterised in that** said at least one blade (3a) is vertical and is rotatably constrained to the base (2c) of said reaction channel (2).

5. Bioreactor according to claim 1, **characterised in that** said detecting means (5) comprise second measuring means (15) arranged on said at least one blade (3a) for detecting over time the hydrodynamic forces acting on said at least one blade, said regulating means (6) dynamically regulating the displacement frequency and/or amplitude of said at least one blade (3a) depending on the information received from said second measuring means (5).

6. Bioreactor according to claim 5, **characterised in that** said second measuring means comprise at least one force sensor.

7. Bioreactor (1) according to claims 5 and 6, **characterised in that** said at least one blade (3a) is wedge-shaped and is constrained in a translatable manner to the base (2c) of said reaction channel (2).

8. Bioreactor (1) according to one or more of claims 1 to 7, **characterised in that** said regulating means (6) comprise at least one control unit (6a) adapted to receive the information received from said first measuring means (5) or from said second measuring means (15) and to process them, said control unit (6a) controlling the displacement frequency and/or amplitude of said at least one blade (3a) depending on the results obtained by said control unit.

9. Bioreactor (1) according to one or more of claims 1 to 8, **characterised in that** said dynamic regulating means (6) comprise at least one motor and/or actuator (11) for the displacement of said at least one blade (3a), said motor and/or actuator being functionally connected to said control unit (6a) to be controlled to transmit the displacement control to said at least one blade according to a frequency and/or amplitude calculated depending on the result obtained by said control unit.

10. Bioreactor (1) according to one or more of claims 1 to 9, **characterised by** comprising one or more filtering elements (20,21) for filtering the signal coming from said first measuring means, or from said second measuring means, to said control unit.

11. Method for producing photosynthetic micro-organisms by means of a bioreactor according to one or more of claims 1 to 10, comprising the step of:
a) generating, by means of said generating means, propagation waves along the longitudinal axis (X) of said reaction channel (2) containing a liquid culture of said micro-organisms, said step a) being carried out starting from a input command signal to said generating means in which the shape of the harmonic gravity waves (102) intended to be generated along the longitudinal axis of said reaction channel, is depicted;
**characterised by** comprising the steps of:
b) detecting the component of the reflected waves which is contained in said longitudinal propagation waves produced by said generating means;
c) dynamically regulating the displacement frequency and/or amplitude of said at least one blade (3a) depending on the information received during the step b) in such a way as to zero out said component of the reflected waves which is contained in said longitudinal propagation waves to obtain the shape of said harmonic gravity waves (102) intended to be generated along the longitudinal axis of said reaction channel (2).

12. Method according to claim 11, **characterised in that** said step b) comprises the step b1) of comparing the shape of said longitudinal propagation waves (101) generated by said generating means (3) in said step a), with the shape of said harmonic gravity waves (102) intended to be generated by said generating means, in such a way that the longitudinal propagation wave generated by said generating means substantially coincides with the wave intended to be generated by said generating means.

13. Method according to claim 12, **characterised in that** said step b1) comprises the step b1a) of detecting the level variations (Y1(t), Y2(t)) of the free surface of said liquid culture, over time, in at least two distinct points (X1, X2) arranged along said longitudinal axis (X) of said reaction channel (2), thus obtaining a plurality of pairs of level variations (Y1(t1), Y2(t1); Y1(t2), Y2(t2);......; Y1(tn), Y2(tn)) at different instants, said two distinct points being measured from a first origin point (X0) of generation of said harmonic gravity waves by said at least one blade (3a), the step b1b) of adding, for each detection carried out over time (t1, t2,..., tn), the two detected level variations, thus obtaining a plurality of sums of level variations (Y1(t1)+Y2(t1); Y1(t2)+Y2(t2);......; Y1(tn)+Y2(tn)), and the step b1c) of subtracting, for each detection, said plurality of sums of such level variations (Y1(t1)+Y2(t1), Y1(t2)+Y2(t2),......, Y1(tn)+Y2(tn)) from the sum of two level variations (Z1(t1)+Z2(t1); Z1(t2)+Z2(t2);......; Z1(tn)+Z2(tn)) of the shape of said harmonic gravity waves intended to be generated, taken in two points (L1, L2) arranged at a distance, from a second origin (L0), equal to that at which said two distinct points (X1, X2) are located with respect to said first origin point (X0) of said propagation waves generated by said at least one blade.

14. Method according to claim 13, **characterised in that** said step c) comprises the step c1) of varying the displacement frequency and/or amplitude of said at least one blade if said difference calculated in said step b1c) is different from zero.

15. Method according to claim 11, **characterised in that** said step b) comprises the step b1') of detecting over time the hydrodynamic forces acting on said at least one blade, and the step b2') of subtracting for each instant the force measured from the force value expected at that given instant; said step c) further comprising the step c1') of varying the displacement frequency and/or amplitude of said at least one blade if said difference calculated in said step b2') is different from zero.

16. Method according to claim 14 or 15, **characterised in that** in said step c1), or c1'), said variation in frequency and/or amplitude is obtained through a transfer function which uses said value calculated in said step b1c), or b2') as input signal, and **in that** it is adapted to feedback minimise said difference value calculated in said step b1c), or b2'), through a closed-circuit control system of the PID type or neural networks or fuzzy logic or the like.

## Patentansprüche

1. Bioreaktor (1) zur Herstellung photosynthetischer Mikroorganismen, umfassend
einen Reaktionskanal (2), der eine Flüssigkultur (100) der Mikroorganismen enthält, und Erzeugungsmittel (3) zum Erzeugen von Wellen (101), die sich in Richtung der Längsachse (X) des Reaktionskanals (2) ausbreiten, ausgehend von einem Eingangsbefehlsignal zu den Erzeugungsmittel, in dem die Form der harmonischen Schwerewellen (102), die entlang der Längsachse (X) des Reaktionskanals (2) zur Erzeugung vorgesehen sind, dargestellt ist, wobei die Erzeugungsmittel (3) mindestens ein innerhalb des Reaktionskanals (2) beweglich angeordnetes Blatt (3a) an einem ersten Ende (2a) des Kanals umfassen, um die längsgerichteten Ausbreitungswellen (101) zu erzeugen, wobei der Bioreaktor (1) **dadurch gekennzeichnet ist, dass** er ferner Erfassungsmittel (5) zum Erfassen der Komponente der reflektierten Wellen umfasst, die in den durch die Erzeugungsmittel produzierten längsgerichteten Ausbreitungswellen enthalten ist, und Regelmittel (6) zur dynamischen Regelung der Verschiebungsfrequenz und/oder Amplitude des mindestens einen Blattes (3a) in Abhängigkeit von den von den Erfassungsmitteln empfangenen Informationen, derart, dass die in den längsgerichteten Ausbreitungswellen (101) enthaltene Komponente der reflektierten Wellen auf Null reduziert wird, um die Form der harmonischen Schwerewellen (102) zu erhalten, die entlang der Längsachse des Reaktionskanals (2) zur Erzeugung vorgesehen sind.

2. Bioreaktor nach Anspruch 1, **dadurch gekennzeichnet, dass** die Erfassungsmittel (5) erste Messmittel (10) umfassen, die innerhalb des Reaktionskanals (2) angeordnet sind, um im Zeitverlauf die Form der durch die Erzeugungsmittel (3) erzeugten längsgerichteten Ausbreitungswellen zu erfassen, wobei die Regelmittel (6) die Verschiebungsfrequenz und/oder Amplitude des mindestens einen Blattes (3a) dynamisch regeln, in Abhängigkeit von den von den ersten Messmitteln (10) empfangenen Informationen, durch Vergleich der Form der durch die Erzeugungsmittel (3) erzeugten und von den ersten Messmitteln (10) erfassten längsgerichteten Ausbreitungswellen (101) mit der Form der harmonischen Schwerewellen (102), die durch die Erzeugungsmittel (3) zur Erzeugung vorgesehen sind, derart, dass die durch die Erzeugungsmittel erzeugte Welle im Wesentlichen mit der Welle übereinstimmt, die durch die Erzeugungsmittel zur Erzeugung vorgesehen ist.

3. Bioreaktor (1) nach Anspruch 2, **dadurch gekennzeichnet, dass** die ersten Messmittel (10) mindestens zwei Pegelsensoren (10a, 10b) umfassen, fähig, im Zeitverlauf die Pegeländerung der innerhalb der Flüssigkultur durch die Erzeugungsmittel (3) erzeugten längsgerichteten Ausbreitungswellen zu messen und diese Informationen an die Regelmittel (6) zu übermitteln, wobei die mindestens zwei Pegelsensoren (10a, 10b) in zwei unterschiedlichen Positionen des Raums (X1, X2) entlang der Längsrichtung des Kanals (X) angeordnet sind.

4. Bioreaktor (1) nach einem oder mehreren der Ansprüche 1 bis 3, **dadurch gekennzeichnet, dass** das mindestens eine Blatt (3a) vertikal und drehbar an der Basis (2c) des Reaktionskanals (2) gelagert ist.

5. Bioreaktor nach Anspruch 1, **dadurch gekennzeichnet, dass** die Erfassungsmittel (5) zweite Messmittel (15) umfassen, die an dem mindestens einen Blatt (3a) angeordnet sind, um im Zeitverlauf die auf das mindestens eine Blatt wirkenden hydrodynamischen Kräfte zu erfassen, wobei die Regelmittel (6) die Verschiebungsfrequenz und/oder Amplitude des mindestens einen Blattes (3a) dynamisch regeln, in Abhängigkeit von den von den zweiten Messmitteln (5) empfangenen Informationen.

6. Bioreaktor nach Anspruch 5, **dadurch gekennzeichnet, dass** die zweiten Messmittel mindestens einen Kraftsensor umfassen.

7. Bioreaktor (1) nach den Ansprüchen 5 und 6, **dadurch gekennzeichnet, dass** das mindestens eine Blatt (3a) keilförmig ist und in übersetzbarer Weise an der Basis (2c) des Reaktionskanals (2) gelagert ist.

8. Bioreaktor (1) nach einem oder mehreren der Ansprüche 1 bis 7, **dadurch gekennzeichnet, dass** die Regelmittel (6) mindestens eine Steuereinheit (6a) umfassen, die dazu angepasst ist, die von den ersten Messmitteln (5) oder von den zweiten Messmitteln (15) empfangenen Informationen zu empfangen und zu verarbeiten, wobei die Steuereinheit (6a) die Verschiebungsfrequenz und/oder Amplitude des mindestens einen Blattes (3a) in Abhängigkeit von den durch die Steuereinheit erzielten Ergebnissen steuert.

9. Bioreaktor (1) nach einem oder mehreren der Ansprüche 1 bis 8, **dadurch gekennzeichnet, dass** die dynamischen Regelmittel (6) mindestens einen Motor und/oder Aktuator (11) zur Verschiebung des mindestens einen Blattes (3a) umfassen, wobei der Motor und/oder Aktuator funktional mit der Steuereinheit (6a) verbunden ist, um gesteuert zu werden, die Steuerung der Verschiebung an das mindestens eine Blatt gemäß einer Frequenz und/oder Amplitude zu übermitteln, die in Abhängigkeit von den durch die Steuereinheit erzielten Ergebnissen berechnet wird.

10. Bioreaktor (1) nach einem oder mehreren der Ansprüche 1 bis 9, **dadurch gekennzeichnet, dass** er ein oder mehrere Filterelemente (20, 21) umfasst, die dazu bestimmt sind, das von den ersten Messmitteln oder von den zweiten Messmitteln kommende Signal an die Steuereinheit zu filtern.

11. Verfahren zur Herstellung photosynthetischer Mikroorganismen mittels eines Bioreaktors nach einem oder mehreren der Ansprüche 1 bis 10, umfassend den Schritt:
a) Erzeugen, mittels der Erzeugungsmittel, von Ausbreitungswellen entlang der Längsachse (X) des Reaktionskanals (2), der eine Flüssigkultur der Mikroorganismen enthält, wobei der Schritt a) ausgehend von einem Eingangsbefehlsignal zu den Erzeugungsmittel durchgeführt wird, in dem die Form der harmonischen Schwerewellen (102), die entlang der Längsachse des Reaktionskanals zur Erzeugung vorgesehen sind, dargestellt ist;
**dadurch gekennzeichnet, dass** das Verfahren ferner die Schritte umfasst:
b) Erfassen der Komponente der reflektierten Wellen, die in den durch die Erzeugungsmittel produzierten längsgerichteten Ausbreitungswellen enthalten ist;
c) dynamisches Regeln der Verschiebungsfrequenz und/oder Amplitude des mindestens einen Blattes (3a) in Abhängigkeit von den während des Schrittes b) empfangenen Informationen, derart, dass die in den längsgerichteten Ausbreitungswellen enthaltene Komponente der reflektierten Wellen auf Null reduziert wird, um die Form der harmonischen Schwerewellen (102) zu erhalten, die entlang der Längsachse des Reaktionskanals (2) zur Erzeugung vorgesehen sind.

12. Verfahren nach Anspruch 11, **dadurch gekennzeichnet, dass** der Schritt b) den Schritt b1) umfasst, bei dem die Form der längsgerichteten Ausbreitungswellen (101), die durch die Erzeugungsmittel (3) in dem Schritt a) erzeugt werden, mit der Form der harmonischen Schwerewellen (102), die durch die Erzeugungsmittel zur Erzeugung vorgesehen sind, verglichen wird, derart, dass die durch die Erzeugungsmittel erzeugte längsgerichtete Ausbreitungswelle im Wesentlichen mit der Welle übereinstimmt, die durch die Erzeugungsmittel zur Erzeugung vorgesehen ist.

13. Verfahren nach Anspruch 12, **dadurch gekennzeichnet, dass** der Schritt b1) umfasst: den Schritt b1a), bei dem die Pegeländerungen (Y1(t), Y2(t)) der freien Oberfläche der Flüssigkultur im Zeitverlauf an mindestens zwei verschiedenen Punkten (X1, X2), die entlang der Längsachse (X) des Reaktionskanals (2) angeordnet sind, erfasst werden, wodurch eine Vielzahl von Paaren von Pegeländerungen (Y1(t1), Y2(t1); Y1(t2), Y2(t2);......; Y1(tn), Y2(tn)) zu verschiedenen Zeitpunkten erhalten wird, wobei die zwei verschiedenen Punkte von einem ersten Ursprungspunkt (X0) der Erzeugung der harmonischen Schwerewellen durch das mindestens eine Blatt (3a) aus gemessen werden, den Schritt b1b) des Addierens, für jede über die Zeit (t1, t2,..., tn) durchgeführte Erfassung, der beiden erfassten Pegeländerungen, wodurch eine Vielzahl von Summen der Pegeländerungen (Y1(t1)+Y2(t1); Y1(t2)+Y2(t2);......; Y1(tn)+Y2(tn)) erhalten wird; und den Schritt b1c) des Subtrahierens, für jede Erfassung, der Vielzahl von Summen derartiger Pegeländerungen (Y1(t1)+Y2(t1), Y1(t2)+Y2(t2),......, Y1(tn)+Y2(tn)) von der Summe zweier Pegeländerungen (Z1(t1)+Z2(t1); Z1(t2)+Z2(t2);......; Z1(tn)+Z2(tn)) der Form der harmonischen Schwerewellen, die zur Erzeugung vorgesehen sind, aufgenommen in zwei Punkten (L1, L2), die in einem Abstand von einem zweiten Ursprungspunkt (L0) angeordnet sind, der demjenigen Abstand entspricht, in dem sich die zwei verschiedenen Punkte (X1, X2) in Bezug auf den ersten Ursprungspunkt (X0) der durch das mindestens eine Blatt erzeugten Ausbreitungswellen befinden.

14. Verfahren nach Anspruch 13, **dadurch gekennzeichnet, dass** der Schritt c) den Schritt c1) umfasst, bei dem die Verschiebungsfrequenz und/oder Amplitude des mindestens einen Blattes geändert wird, falls die in dem Schritt b1c) berechnete Differenz ungleich Null ist.

15. Verfahren nach Anspruch 11, **dadurch gekennzeichnet, dass** der Schritt b) den Schritt b1') umfasst, bei dem im Zeitverlauf die auf das mindestens eine Blatt wirkenden hydrodynamischen Kräfte erfasst werden, und den Schritt b2'), bei dem für jeden Zeitpunkt die gemessene Kraft von dem für diesen bestimmten Zeitpunkt erwarteten Kraftwert subtrahiert wird; wobei der Schritt c) ferner den Schritt c1') umfasst, bei dem die Verschiebungsfrequenz und/oder Amplitude des mindestens einen Blattes geändert wird, falls die in dem Schritt b2') berechnete Differenz ungleich Null ist.

16. Verfahren nach Anspruch 14 oder 15, **dadurch gekennzeichnet, dass** in dem Schritt c1) oder c1') die Frequenz- und/oder Amplitudenänderung durch eine Übertragungsfunktion erfolgt, die den in dem Schritt b1c) oder b2') berechneten Wert als Eingangssignal verwendet und die dazu angepasst ist, den in dem Schritt b1c) oder b2') berechneten Differenzwert rückgekoppelt zu minimieren, mittels eines geschlossenen Regelkreissystems vom Typ PID oder neuronale Netze oder Fuzzy-Logik oder dergleichen.

## Revendications

1. Bioréacteur (1) pour la production de micro-organismes photosynthétiques, comprenant un canal de réaction (2) contenant une culture liquide (100) desdits micro-organismes et des moyens de génération (3) pour générer des ondes (101) qui se propagent dans la direction de l'axe longitudinal (X) dudit canal de réaction (2), à partir d'un signal de commande d'entrée vers lesdits moyens de génération dans lesquels la forme des ondes de gravité harmonique (102) destinées à être générées le long de l'axe longitudinal (X) dudit canal de réaction (2) est représentée, lesdits moyen de génération (3) comprenant au moins une lame (3a) contrainte de façon mobile à l'intérieur dudit canal de réaction (2), au niveau d'une première extrémité dudit canal (2a), pour générer lesdites ondes de propagation longitudinale (101), ledit bioréacteur (1) étant **caractérisé en ce qu'**il comprend également des moyens de détection (5) pour détecter la composante des ondes réfléchies qui sont contenues dans lesdites ondes de propagation longitudinale produites par lesdits moyens de génération, et des moyens de régulation (6) pour réguler dynamiquement la fréquence et/ou l'amplitude de déplacement de ladite au moins une lame (3a) en fonction des informations reçues desdits moyens de détection, de manière à annuler la composante des ondes réfléchies qui est contenue dans lesdites ondes de propagation longitudinale (101) pour obtenir la forme desdites ondes de gravité harmonique (102) destinées à être générées le long de l'axe longitudinal dudit canal de réaction (2).

2. Bioréacteur selon la revendication 1, **caractérisé en ce que** lesdits moyens de détection (5) comprennent des premiers moyens de mesure (10) disposés à l'intérieur dudit canal de réaction (2) pour détecter au cours du temps la forme des ondes de propagation longitudinale générées par lesdits moyens de génération (3), lesdits moyens de régulation (6) régulent dynamiquement la fréquence et/ou l'amplitude de déplacement de ladite au moins une lame (3a) en fonction des informations reçues desdits premiers moyens de mesure (10) en comparant la forme desdites ondes de propagation longitudinale (101) générées par lesdits moyens de génération (3) et détectées par lesdits premiers moyens de mesure (10), avec la forme desdites ondes de gravité harmoniques (102) destinées à être générées par lesdits moyens de génération (3), de telle sorte que l'onde générée par lesdits moyens de génération coïncide substantiellement avec l'onde destinée à être générée par lesdits moyens de génération.

3. Bioréacteur (1) selon la revendication 2, **caractérisé en ce que** lesdits premiers moyens de mesure (10) comprennent au moins deux capteurs de niveau (10a, 10b) aptes à mesurer au cours du temps la variation de niveau desdites ondes de propagation longitudinale générées par lesdits moyens de génération (3) au sein de ladite culture liquide, et à envoyer lesdites informations auxdits moyens de régulation (6), lesdits au moins deux capteurs de niveau (10a, 10b) étant disposés en deux positions distinctes de l'espace (X1 , X2) le long de ladite direction longitudinale dudit canal (X).

4. Bioréacteur (1) selon une ou plusieurs des revendications 1 à 3, **caractérisé en ce que** ladite au moins une lame (3a) est verticale et est contrainte en rotation à la base (2c) dudit canal de réaction (2).

5. Bioréacteur selon la revendication 1, **caractérisé en ce que** lesdits moyens de détection (5) comprennent des seconds moyens de mesure (15) disposés sur ladite au moins une lame (3a) pour détecter au cours du temps les forces hydrodynamiques agissant sur ladite au moins une lame, lesdits moyens de régulation (6) régulant dynamiquement la fréquence et/ou l'amplitude de déplacement de ladite au moins une lame (3a) en fonction des informations reçues desdits seconds moyens de mesure (5).

6. Bioréacteur selon la revendication 5, **caractérisé en ce que** lesdits seconds moyens de mesure comprennent au moins un capteur de force.

7. Bioréacteur (1) selon les revendications 5 et 6, **caractérisé en ce que** ladite au moins une lame (3a) est cunéiforme et est contrainte en translation à la base (2c) dudit canal de réaction (2).

8. Bioréacteur (1) selon une ou plusieurs des revendications 1 à 7, **caractérisé en ce que** lesdits moyens de régulation (6) comprennent au moins une unité de contrôle (6a) apte à recevoir les informations reçues desdits premiers moyens de mesure (5) ou desdits seconds moyens de mesure (15) et à les traiter, ladite unité de contrôle (6a) contrôlant la fréquence et/ou l'amplitude de déplacement de ladite au moins une lame (3a) en fonction des résultats obtenus par ladite unité de contrôle.

9. Bioréacteur (1) selon une ou plusieurs des revendications 1 à 8, **caractérisé en ce que** lesdits moyens de régulation dynamique (6) comprennent au moins un moteur et/ou un actionneur (11) de déplacement de ladite au moins une lame (3a), ledit moteur et/ou actionneur étant relié fonctionnellement à ladite unité de contrôle (6a) pour être contrôlé afin de transmettre le contrôle de déplacement à ladite au moins une lame selon une fréquence et/ou une amplitude calculée en fonction du résultat obtenu par ladite unité de contrôle.

10. Bioréacteur (1) selon l'une ou plusieurs des revendications 1 à 9, **caractérisé en ce qu'**il comprend un ou plusieurs éléments de filtrage (20,21) pour filtrer le signal provenant desdits premiers moyens de mesure, ou desdits seconds moyens de mesure, vers ladite unité de contrôle.

11. Procédé de production de micro-organismes photosynthétiques au moyen d'un bioréacteur selon l'une ou plusieurs des revendications 1 à 10, comprenant l'étape consistant à :
a) générer, au moyen desdits moyens de génération, des ondes de propagation le long de l'axe longitudinal (X) dudit canal de réaction (2) contenant une culture liquide desdits micro-organismes, ladite étape a) étant réalisée à partir d'un signal de commande d'entrée vers lesdits moyens de génération dans lesquels la forme des ondes de gravité harmonique (102) destinées à être générées le long de l'axe longitudinal dudit canal de réaction est représentée ;
**caractérisé en ce qu'**il comprend les étapes consistant à :
b) détecter la composante des ondes réfléchies qui est contenue dans lesdites ondes de propagation longitudinales produites par lesdits moyens de génération ;
c) réguler dynamiquement la fréquence et/ou l'amplitude de déplacement de ladite au moins une lame (3a) en fonction des informations reçues lors de l'étape b) de manière à annuler la composante des ondes réfléchies qui est contenue dans lesdites ondes de propagation longitudinale pour obtenir la forme desdites ondes de gravité harmonique (102) destinées à être générées le long de l'axe longitudinal dudit canal de réaction (2).

12. Procédé selon la revendication 11, **caractérisé en ce que** ladite étape b) comprend l'étape b1) consistant à comparer la forme desdites ondes de propagation longitudinale (101) générées par lesdits moyens de génération (3) dans ladite étape a), avec la forme desdites ondes de gravité harmonique (102) destinées à être générées par lesdits moyens de génération, de telle sorte que l'onde de propagation longitudinale générée par lesdits moyens de génération coïncide substantiellement avec l'onde destinée à être générée par lesdits moyens de génération.

13. Procédé selon la revendication 12, **caractérisé en ce que** ladite étape b1) comprend l'étape b1a) consistant à détecter les variations de niveau (Y1(t), Y2(t)) de la surface libre de ladite culture liquide, au cours du temps, en au moins deux points distincts (X1, X2) disposés le long dudit axe longitudinal (X) dudit canal de réaction (2), obtenant ainsi une pluralité de paires de variations de niveau (Y1(t1), Y2(t1) ; Y1(t2), Y2(t2) ;.....; Y1(tn), Y2(tn)) à des instants différents, lesdits deux points distincts étant mesurés à partir d'un premier point d'origine (X0) de génération desdites ondes de gravité harmonique par ladite au moins une lame (3a), l'étape b1b) consistant à additionner, pour chaque détection effectuée au cours du temps (t1, t2,.. tn), les deux variations de niveau détectées, obtenant ainsi une pluralité de sommes de variations de niveau (Y1(t1)+Y2(t1) ; Y1(t2)+Y2(t2) ;.....; Y1(tn)+Y2(tn)), et l'étape b1c) consistant à soustraire, pour chaque détection, ladite pluralité de sommes de telles variations de niveau (Y1(t1)+Y2(t1), Y1(t2)+Y2(t2),.....,Y1(tn)+Y2(tn)) de la somme de deux variations de niveau (Z1(t1)+Z2(t1) ; Z1(t2)+Z2(t2) ;.....; Z1(tn)+Z2(tn)) de la forme desdites ondes de gravité harmonique destinées à être générées, prises en deux points (L1, L2) disposés à une distance, à partir d'une seconde origine (L0), égale à celle à laquelle se situent lesdits deux points distincts (X1, X2) par rapport audit premier point d'origine (X0) desdites ondes de propagation générées par ladite au moins une lame.

14. Procédé selon la revendication 13, **caractérisé en ce que** ladite étape c) comprend l'étape c1) de faire varier la fréquence et/ou l'amplitude de déplacement de ladite au moins une lame si ladite différence calculée à ladite étape b1c) est différente de zéro.

15. Procédé selon la revendication 11, **caractérisé en ce que** ladite étape b) comprend l'étape b1') consistant à détecter au cours du temps des forces hydrodynamiques agissant sur ladite au moins une lame, et l'étape b2') consistant à soustraire pour chaque instant de la force mesurée de la valeur de force attendue à cet instant donné ; ladite étape c) comprend en outre l'étape c1') consistant à varier la fréquence et/ou de l'amplitude de déplacement de ladite au moins une lame si ladite différence calculée dans ladite étape b2') est différente de zéro.

16. Procédé selon la revendication 14 ou 15, **caractérisé en ce que** dans ladite étape c1), ou c1'), ladite variation de fréquence et/ou d'amplitude est obtenue par une fonction de transfert qui utilise ladite valeur calculée dans ladite étape b1c), ou b2') comme signal d'entrée, et **en ce qu'**elle est adaptée pour minimiser en retour ladite valeur de différence calculée dans ladite étape b1c), ou b2'), par un système de contrôle en circuit fermé du type PID ou réseaux neuronaux ou logique floue ou similaire.
